(19) Europäisches Patentamt / European Patent Office / Office européen des brevets

(11) **EP 3 469 646 B1**

(12) **EUROPEAN PATENT SPECIFICATION**

(45) Date of publication and mention
of the grant of the patent:
**20.01.2021 Bulletin 2021/03**

(21) Application number: **17730250.2**

(22) Date of filing: **09.06.2017**

(51) Int Cl.:
*H01M 8/0206* (2016.01)     *H01M 8/0213* (2016.01)
*H01M 8/0228* (2016.01)     *H01M 4/62* (2006.01)
*H01M 4/66* (2006.01)     *H01M 8/1018* (2016.01)
*C07D 235/04* (2006.01)     *C07D 249/18* (2006.01)

(86) International application number:
**PCT/GB2017/051697**

(87) International publication number:
**WO 2017/212295 (14.12.2017 Gazette 2017/50)**

(54) **CORROSION PROTECTION COATING**

KORROSIONSSCHUTZBESCHICHTUNG

REVÊTEMENT DE PROTECTION CONTRE LA CORROSION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.06.2016 GB 201610144**

(43) Date of publication of application:
**17.04.2019 Bulletin 2019/16**

(73) Proprietor: **IP2IPO Innovations Limited
London EC4N 8AF (GB)**

(72) Inventors:
• **KUCERNAK, Anthony Robert John
London SW7 2PG (GB)**
• **LAPINSKI, Jacek
Swindon
SN5 8PF (GB)**

(74) Representative: **TLIP Limited
14 King Street
Leeds LS1 2HL (GB)**

(56) References cited:
**US-A1- 2005 126 429     US-A1- 2009 061 184
US-A1- 2010 193 573**

## Description

## Field of the Invention

This invention relates to an electrically conductive composite coating for use in providing a corrosion resistant coating for components in electrochemical devices and a method of coating a component therewith.

## Background to the Invention

In the environment of fuel cells, particularly flexiplanar proton exchange membrane (PEM) fuel cells, metals, such as copper or stainless steel, appear to corrode rapidly over relatively short period of time and, as a result, the overall operational lifetime of the fuel cell is short. However, these metals have some benefits - they have high electrical and thermal conductivity, and are easy to produce complicated structures with, as is performed within the printed circuit board (PCB) industry using copper as a conductor. Corrosion is detrimental to fuel cell operation for a number of reasons: for instance it releases soluble metal ions which deactivate catalysts and impair operation of the electrolyte or the corrosion products result in an insulating barrier which prevents proper current flow.

Flexi-planar PEM fuel cell technology employs PCB to manufacture the fuel cells. One of the components is a planar plate commonly known as a current collector. In general, the current collector can have a metallic structure, for instance specifically shaped stainless steel or even a single metallic layer (e.g. copper) on a composite board.

Conditions in the fuel cell can also vary with time, for example, pH may vary from 1 to 6 (more extreme when in contact with the ion conducting membrane) as well as concentration of ions due to the dissolution of the membrane (e.g. $F^-$ or organic species). Moreover, the change in temperature is an unavoidable process, especially during the start-up, operation and shut down of the fuel cell. Consequently, any component in the fuel cell is exposed to rather unstable conditions.

Thus, there is a need to provide a protective coating that must withstand these unstable conditions protecting the components from corrosion. Compactness of the coating is also of utmost importance.

In addition to that the coating must have very good electrical conductivity in order to minimise IR drop during the fuel cell operation. The U.S. Department of Energy (DoE) specifies the optimum performance of coated bipolar plates in fuel cells as having corrosion resistance with corrosion current less than 1 $\mu Acm^{-2}$ for the cathode and anode side and a conductivity of greater than 100 $Scm^{-1}$. Furthermore, they set the contact resistance goal for PEM fuel cell applications at lower than 10 $m\Omega$ $cm^2$ at 140 N/cm$^2$ compaction pressure for 2015 (Wind J, Spah R, Kaiser W, Böhm G. Metallic bipolar plates for PEM fuel cells. J Power Sources 2002;105:256-260. These requirements pose a rather large demand on the performance of the coating. Further prior art relating to corrosion protection coatings can be found in US 2009/061184 A1, US 2010/193573 A1 and US 2005/126429 A1.

Thus, there is a need for an improved coating for substrates and components used in fuel cells. Similarly, there is need for improved corrosion protection coatings in, for example, batteries, redox flow batteries, electrolysers and supercapacitors.

## Summary of the Invention

The corrosion potential of fuel cells resulting from the reactant materials used can be significantly reduced by applying appropriate coatings to components of the fuel cell. The invention provides a coating that can be applied to a substrate to reduce the corrosion rate to a minimum and maintain high electrical conduction and low contact resistance. Accordingly, in a first aspect, the invention provides an electrically conductive composite coating comprising:

a layer comprising an electrically conductive coating material comprising a carbon-based material and an azole-containing corrosion inhibitor; and
a layer of tin and/or a binary or ternary alloy of tin with one or more of nickel, antimony, indium or gallium, said tin layer being directly deposited on the substrate and having the electrically conductive coating on top of it, according to independent claim 1.

The following discussion of the layer comprising an electrically conductive coating material, the carbon-based material, the azole-containing corrosion inhibitor and the layer comprising tin in relation to the first aspect of the invention apply *mutatis mutandis* to the second to the eighth aspects of the invention which follow.

The carbon-based material may comprise carbon black, coal, charcoal, graphite, carbon fibres, carbon nanotubes or graphene or mixtures thereof.

The electrically conductive coating material may further comprise an organic binder. Preferably the organic binder comprises organic monomeric and/or oligomeric polymer precursor compounds. The electrically conductive coating material may comprise a carbon ink comprising the carbon-based material and the organic binder, so that the carbon-based material and organic binder are components of the carbon ink. The electrically conductive coating material may comprise about 50 wt% to about 90 wt% of the carbon-based material, preferably about 70 to about 90 wt% or about 80 wt% of the carbon-based material.

The azole-containing corrosion inhibitor may be benzotriazole, 2-mercaptobenzimidazole, 5-phenyl tetrazole, *bis*[4-amino-5-hydroxy-1,2,4-triazol-3-yl] methane or *bis*[4-amino-5-hydroxy-1,2,4-triazol-3-yl]butane, or mixtures thereof, preferably the azole-containing corrosion inhibitor is benzotriazole. The azole-containing corrosion inhibitor may be present in the electrically conductive coating material in an amount of about 10 wt% or less of the amount of carbon-based material and organic binder, preferably about 5 wt% or less, preferably about 1 wt%. When the carbon-based material and organic binder are provided as components in a carbon ink, the azole-containing corrosion inhibitor may be present in the electrically conductive coating material in an amount of about 10 wt% or less of the amount of carbon ink, preferably about 5 wt% or less, preferably about 1 wt%.

The layer comprising tin may comprise tin metal and/or tin alloy, for example, a tin-nickel alloy, a tin-antimony alloy, a tin-indium alloy, a tin-gallium alloy, a tin-indium-antimony alloy or tin-nickel-antimony alloy or mixtures thereof.

The layer comprising an electrically conductive coating material may be about 1 to about 100 $\mu$m thick, preferably about 1 to about 50 $\mu$m thick, preferably about 1 to about 40 $\mu$m thick, about 5 to about 30 $\mu$m thick, about 5 to about 25 $\mu$m thick, about 10 to about 20 $\mu$m thick, or about 15 to about 20 $\mu$m thick. The layer comprising tin may be about 1 to about 20 $\mu$m thick, preferably about 2 to about 15 $\mu$m thick, or about 5 to about 10 $\mu$m thick.

The electrically conductive composite coating may have an area specific electrical resistance of less than about 10 m$\Omega$ cm$^2$.

The electrically conductive composite coating material as described herein may be for use in coating a substrate, for example, a metallic substrate, preferably a copper, iron, titanium, aluminium, nickel or stainless steel substrate. The substrate may be a component in an electrochemical device, such as a fuel cell assembly, a battery, a redox flow battery, an electrolyser or a supercapacitor.

In a second aspect, the invention provides a coated article comprising;

a substrate as described herein; and
an electrically conducting composite coating comprising a layer comprising an electrically conductive coating material comprising a carbon-based material and an azole-containing corrosion inhibitor, and a layer comprising tin.

The coated article may comprise an electrically conducting composite coating comprising:

two or more layers comprising an electrically conductive coating material comprising a carbon-based material and an azole-containing corrosion inhibitor; and
two or more layers comprising tin.

The electrically conducting composite coating may comprise any of the features discussed in relation to the first aspect of the invention.

The substrate may be a metallic substrate, preferably a copper, iron, titanium, aluminium, nickel or stainless steel substrate. The substrate may, in particular, be a component for an electrochemical device, such as a fuel cell assembly, a battery, a redox flow battery, an electrolyser or a supercapacitor.

In a third aspect, the invention provides an electrically conductive coating material comprising:

a carbon ink as described herein; and
an azole-containing corrosion inhibitor.

In a fourth aspect, the invention provides an electrically conductive coating material comprising:

a carbon-based material as described herein; and
an azole-containing corrosion inhibitor as described herein, wherein the azole-containing corrosion inhibitor is benzotriazole, 2-mercaptobenzimidazole, 5-phenyl tetrazole, *bis*[4-amino-5-hydroxy-1,2,4-triazol-3-yl]methane or *bis*[4-amino-5-hydroxy-1,2,4-triazol-3-yl]butane, or mixtures thereof, preferably the azole-containing corrosion inhibitor is benzotriazole.

In a fifth aspect, the invention provides a process for coating a substrate with an electrically conductive composite coating, the process comprising:

providing a substrate;
depositing a layer comprising tin on a surface of the substrate; and
depositing a layer comprising an electrically conductive coating material comprising a carbon-based material and an azole-containing corrosion inhibitor as a layer on said surface of the substrate.

The process may further comprise:

depositing a further layer comprising tin on a surface of the coated substrate;
depositing a further layer comprising an electrically conductive coating material comprising a carbon-based material and an azole-containing corrosion inhibitor on said surface of the substrate.

The process may further comprise the step of heat treating the coated substrate after deposition of the layer comprising an electrically conductive coating material, preferably wherein the heat treatment comprises heating to at least about 100°C, at least about 125°C or at least about 150°C, for at least about 30 minutes, at least about 45 minutes or at least about 1 hour.

Depositing a layer comprising tin may comprise electrodeposition of tin and/or a tin alloy onto the substrate. Depositing a layer comprising an electrically conductive coating material may comprise screen printing the electrically conductive coating material as a layer on top of the layer comprising tin.

In a sixth aspect, the invention provides a coated article obtainable by or obtained by the process for coating a substrate with an electrically conductive coating as described herein.

In a seventh aspect, the invention provides an electrochemical device, such as a fuel cell assembly, a battery, a redox flow battery, an electrolyser or a supercapacitor comprising a coated article as described herein.

In an eighth aspect, the invention provides use of an electrically conductive composite coating comprising:

a layer comprising an electrically conductive coating material comprising a carbon-based material and an azole-containing corrosion inhibitor; and a layer comprising tin;
as a corrosion resistant coating for a component in an electrochemical device.

Embodiments described herein in relation to the first and second aspects of the invention apply *mutatis mutandis* to the third to the eighth aspects of the invention.

## Description of Figures

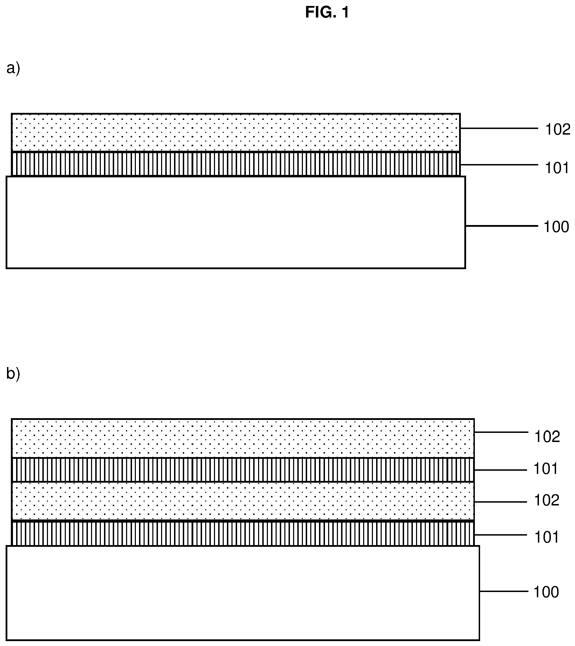

**Figure 1** shows cross sections of (a) a coated article according to the invention comprising a substrate (100) and an electrically conductive composite coating comprising a layer comprising tin (101), provided on at least a surface of the substrate, and a layer comprising an electrically conductive coating material (102), provided on the layer comprising tin; and (b) a coated article according to the invention comprising a substrate (100), two layers comprising an electrically conductive coating material (102), and two layers comprising tin (101).

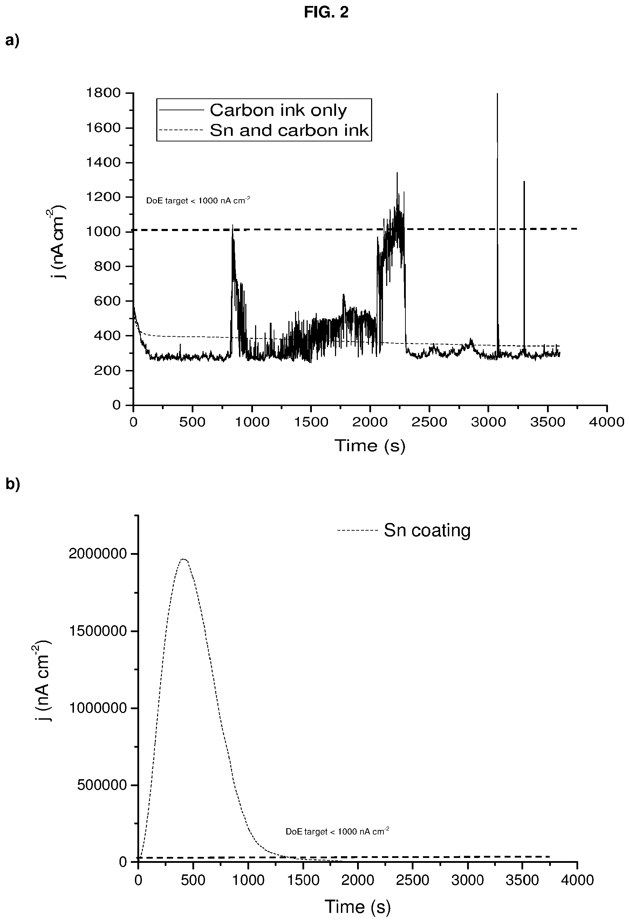

**Figure 2** shows the corrosion current for (a) an electrically conductive coating material layer (solid curve) and an electrically conductive composite coating (dashed curve) and (b) a layer comprising tin. The DoE target of 1000 nA cm$^{-2}$ for corrosion of bipolar plates is shown as a dashed line.

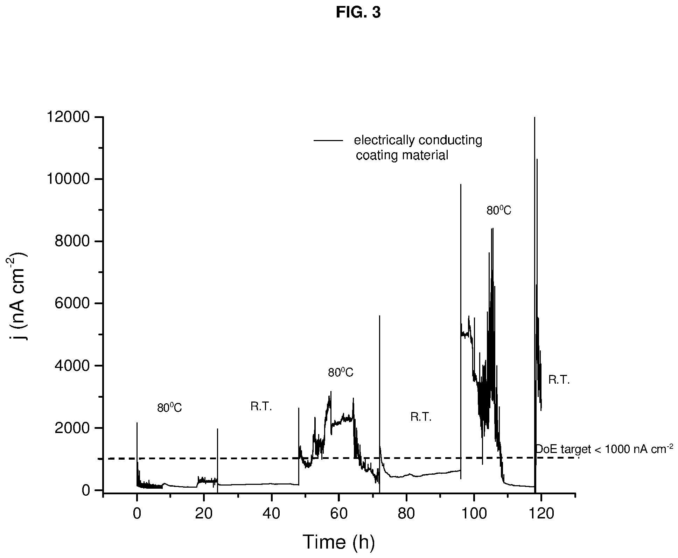

**Figure 3** shows the behaviour of electrically conductive coating material layer in a corrosive environment as part of an accelerated long-term corrosion test. The temperature of the solution was cycled between room temperature (R.T.) and 80°C every 24 h. The DoE target of 1000 nA cm$^{-2}$ for corrosion of bipolar plates is shown as a dashed line.

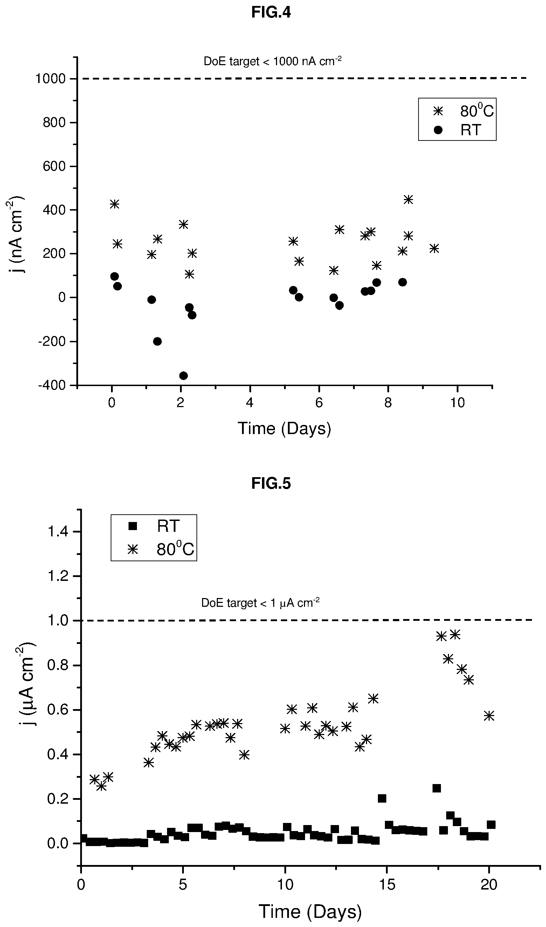

**Figure 4** shows the behaviour of an electrically conductive composite coating in a corrosive medium as part of an accelerated long-term corrosion test. The temperature of the solution was cycled between R.T.

and 80°C every 2 h. The DoE target of 1000 nA cm$^{-2}$ for corrosion of bipolar plates is shown as a dashed line.

**Figure 5** shows the behaviour of a multi-layered electrically conductive composite coating in a corrosive medium as part of an accelerated long-term corrosion test. The coating is composed of four layers: a layer comprising tin as a first layer, an electrically conductive coating material layer as a second layer, a layer comprising a Sn-Sb alloy as a third layer and an electrically conductive coating material layer as a fourth layer. The DoE target of 1000 nA cm$^{-2}$ for corrosion of bipolar plates is shown as a dashed line.

## Detailed Description of the Invention

The corrosion potential of fuel cells, batteries, redox flow batteries, electrolysers and supercapacitors resulting from the reactant materials used can be significantly reduced by applying appropriate coatings to components of a fuel cell, battery, redox flow battery, electrolyser or supercapacitor. Accordingly, in a first aspect, the invention provides electrically conductive composite coating comprising: a layer comprising an electrically conductive coating material comprising a carbon-based material and an azole-containing corrosion inhibitor; and a layer comprising tin. This electrically conductive coating composite may be applied to a surface of a substrate to provide a corrosion resistant layer, whilst maintaining conductivity of the substrate. This composite coating may be applied to components in a fuel cell. Using this approach it has been found that the stability of the components is significantly increased whilst at the same time maintaining a low contact resistance.

The coating composites and materials of the present invention may be applied to a substrate to provide a corrosion resistant protective coating. The substrate may be a metallic substrate in need of corrosion protection, preferably a copper, iron, titanium, aluminium, nickel or stainless steel substrate. The substrate may be a component in a fuel cell, battery, redox flow battery, electrolyser or supercapacitor where the conditions may be harsh and variable during the operation of the device with a lifetime of, for example, 5,000 hours or more. For example, the substrate may be a planar plate in the flexiplanar proton exchange membrane (PEM) fuel cell, as described in patent number' WO 2013/164639.

The flexi-planar technology employs printed circuit boards (PCB) to manufacture the fuel cells. An exemplary component to which the composite coating or coating material of the invention may be applied is a planar plate, commonly known as a current collector.

The coating composites and materials of the present invention provide may be applied to the entirety of the substrate or a part of the substrate. A substrate may have multiple faces. The coating may be applied to coat a single face entirely or more than one of the faces entirely. Alternatively, all of the faces of a substrate may be coated entirely so that the entirety of the substrate is coated.

The present invention relates to electrically conductive composite coatings and coating materials. These coating composites and materials may be used to coat components in a fuel cell, battery, redox flow battery, electrolyser or supercapacitor. Good electrical conductivity is beneficial to minimise voltage drop (IR-drop) during the fuel cell operation. Electrical conductivity is a measure of a material's ability to conduct an electric current. Resistivity is the reciprocal of conductivity and quantifies how strongly a given material opposes the flow of electric current. A low resistivity indicates a material that readily allows the movement of electric charge. The area specific electrical resistance of the electrically conductive composite coating (including the contact resistance) may be less than about 10 m$\Omega$ cm$^2$, wherein the area specific electrical resistance is a product of the resistivity and the thickness of the coating. For example, the electrically conductive composite may have a thickness of about 100 $\mu$m, in which case, the coating may have a resistivity of less than about 1 $\Omega$cm. The electrically conductive composite coating may be less than about 100 $\mu$m thick, preferably about 5 to about 50 $\mu$m, about 5 to about 40 $\mu$m, about 10 to about 30 $\mu$m, about 10 to about 20 $\mu$m. The layer comprising an electrically conductive coating material may be about 1 to about 50 $\mu$m thick, preferably about 5 to about 25 $\mu$m thick, preferably, about 10 to about 20 $\mu$m thick. The layer comprising tin may be about 1 to about 20 $\mu$m thick, preferably about 5 to about 10 $\mu$m thick.

The resistivity (in $\Omega$cm) of the coating composites and materials of the present invention can be calculated from the contact area (in cm$^2$), sample thickness (cm), and resistance ($\Omega$) of a sample according to the following equation:

$$\text{Resistivity} = \text{Resistance} \times \frac{\text{contact area}}{\text{sample thickness}}$$

Resistance is determined by applying a current source to the sample, measuring the resultant voltage, and calculating resistance with Ohm's Law (V = IR). The resistance of the sample may be measured at pressures of about 140 N/cm$^2$ (about 1.4 MPa), which is in accordance with the DoE target for the pressure exerted in the fuel cells. The resistance may then be used to calculate the resistivity, according to the above equation using the contact area and thickness of the sample, and then the area specific electrical resistance, by determining the product of the resistivity and the sample thickness. Measuring of the resistance and resistivity of a coated substrate is described in L. Landis et al., *Making Better Fuel Cells: Through-Plane Resistivity Measurement of Graphite-Filled Bipolar Plates*, 2002, Keithly Instruments, Inc. White Paper (https://www.keithly.co.uk/data?asset=10382).

A carbon-based material of the present invention is a material comprising an elemental form of carbon. An elemental form of carbon is carbon which is at least 90% pure carbon, preferably at least 92%, at least 94%, at least 96%, or at least 98% pure carbon, calculated as a weight percentage. Examples of elemental form of carbon include graphite, graphene, carbon nanotubes, carbon nanofibres, charcoal, coal and carbon black, or mixtures thereof. A carbon-based material of the present invention may consist essentially of an elemental form of carbon. Accordingly, a carbon-based material may consist essentially of graphite, graphene, carbon nanotubes, carbon nanofibres, charcoal, coal and carbon black or mixtures thereof and the material may, therefore, be at least at least 90wt% carbon, preferably at least 92wt%, at least 94wt%, at least 96wt%, or at least 98wt% carbon. A carbon-based material of the present invention may comprise particles of the elemental carbon material.

An electrically conductive coating material of the present invention may further comprise an organic binder. An organic binder serves as a vehicle to hold the particles of the elemental carbon and may be formed from resins or organic monomeric and/or oligomeric polymer precursor compounds. Examples of such resins and precursor compounds include acrylics, alkyds, cellulosic derivatives, rubber resins, ketones, maleics, formaldehydes, phenolics, epoxies, fumarics, hydrocarbons, isocyanate free polyurethanes, poly vinyl butyral, polyamides, shellac, and polycarbonates.

An oligomeric polymer precursor compound may refer to an organic compound comprising 2 or more monomeric repeat units, preferably 2 to 5. A polymer may consist of multiple repeat units, preferably at least 6, and may be a homopolymer, copolymer, or a mixture thereof.

A carbon-based material may be provided as a component in a carbon ink. A carbon ink is a material comprising a carbon-based material and an organic binder. A carbon ink as described herein may preferably be a conductive carbon ink. A carbon ink as described herein is in line with the general understanding in the art. Generally, a carbon ink is an ink that may be easily screen printed and comprises carbon as a pigment along with a binder. A carbon ink may have a dynamic viscosity of between about 2.9 poise and about 7 poise, when calculated at 20°C and 1 atmosphere. Therefore, a carbon ink may be a viscous material. Dynamic viscosity may be measured by a viscometer, calibrated to the viscosity of distilled water at 20°C and 1 atmosphere (0.01 poise).

When a carbon-based material is provided as a component in a carbon ink, the organic binder comprises the resins that are also used to form carbon inks. Exemplary carbon inks include PCB1 Sunchemical ink 2sp, PCB3 Sunchemical ink 2sp revisited and PCB4 Sunchemical ink (last) 2sp.

Azole-containing corrosion inhibitors of the present invention are compounds that contain one or more substituted or unsubstituted, fused or non-fused azole ring. An azole is a class of five-membered nitrogen-containing heterocyclic ring compounds containing at least one other non-carbon ring atom of either nitrogen, sulfur, or oxygen. Exemplary azoles include pyrrole, pyrazole, imidazole, triazole, tetrazole, pentazole, oxazole, isoxazole, thiazole, and isothiazole, optionally substituted with one or more of halo, aliphatic (e.g. alkyl, alkenyl or alkynyl), cycloaliphatic, heterocycloaliphatic, aryl, heteroaryl, alkoxy, amino, hydroxy, carboxyl, acetyl, nitro, cyano, sulfonyl and thiol or combinations thereof. These azole rings may be fused with another ring to form ring system comprising two or more rings. For example, an azole may be fused with an aryl ring (for example, a phenyl ring). The azole-containing corrosion inhibitors of the present invention are chemical compounds that decreases the corrosion rate of a material. Exemplary azole-containing corrosion inhibitors include benzotriazole, 2-mercaptobenzimidazole, 5-phynyl tetrazole, *bis*[4-amino-5-hydroxy-1,2,4-triazol-3-yl]methane or *bis*[4-amino-5-hydroxy-1,2,4-triazol-3-yl]butane, or mixtures thereof, preferably the azole-containing corrosion inhibitor is benzotriazole.

The azole-containing corrosion inhibitor may be present in the electrically conductive coating material at an amount of about 10 weight percent (wt%) or less of the amount of carbon-based material and organic binder, preferably about 5 wt% or less, preferably about 1 wt%. That is to say, the wt% of the corrosion inhibitor is calculated as a percentage of the total weight of carbon-based material and organic binder in the electrically conductive coating material.

An electrically conductive coating material of the present invention may consist essentially of a carbon-based material, organic binder and azole-containing corrosion inhibitor.

An electrically conductive coating material of the present invention may comprise about 50 weight percent (wt%) to about 90 wt% of the carbon-based material, preferably about 60 to about 90 wt%, preferably about 70 to about 90 wt%, about 75 to about 85 wt% or about 80 wt% of the carbon-based material. The remaining part of the electrically conductive coating material may comprise (or consist essentially of) organic binder and azole-containing corrosion inhibitor.

Tin, as used herein, refers to a material comprising tin as a pure metal and/or as a tin-alloy. For example, a tin alloy may be a binary tin alloy or a ternary tin alloy comprising tin and one or more of nickel, antimony, indium or gallium. A tin-alloy is preferably a tin-nickel alloy, a tin-antimony alloy, a tin-indium alloy, a tin-gallium alloy, a tin-indium-antimony alloy or tin-nickel-antimony alloy or mixtures thereof. A layer of tin, as described herein, consists essentially of tin metal and/or a tin alloy as described herein.

In another aspect, the invention provides a process for coating a substrate with an electrically conductive composite coating, the process comprising:

providing a substrate as described herein;

depositing a layer comprising tin on a surface of the substrate; and
depositing a layer comprising an electrically conductive coating material as described herein on said surface of the substrate.

Figure 1 (a) shows a cross section a substrate that has been coated according to a process for coating a substrate as described herein. This coated article formed by this process comprises the substrate (100) and an electrically conductive composite coating comprising the layer comprising tin (101) and the layer comprising an electrically conductive coating material (102).

The steps of depositing a layer comprising tin and depositing a layer comprising an electrically conductive coating material may be repeated one or more times to form a coated substrate comprising an electrically conductive composite coating comprising at least two layers comprising tin and/or at least two layers comprising an electrically conductive coating material. Figure 1 (b) shows a coated article formed by a process for coating a substrate as described herein and further comprising the further repeated steps depositing a layer comprising tin and depositing a layer comprising an electrically conductive coating material. This coated article comprises a substrate (100) and an electrically conductive composite coating comprising two layers comprising an electrically conductive coating material (102), and two layers comprising tin (101).

A coated article, as described herein, comprises a substrate and an electrically conductive coating material comprising a layer comprising an electrically conductive coating material and a layer comprising tin. The layer of tin and/or a binary or ternary alloy of tin with one or more of nickel, antimony, indium or gallium is provided on (preferably directly in contact with) a surface of the substrate. The layer comprising an electrically conductive coating materialis then provided on (preferably directly in contact with) the layer comprising tin so that the layer comprising tin is interposed between the substrate and the layer comprising an electrically conductive coating material, according to independent claims 1 and 14 and as shown in figure 1 (a). A coated article comprising a substrate and an electrically conductive coating material comprising more than one layer (for example, two layers) comprising an electrically conductive coating material and more than one layer (for example, two layers) comprising tin may be structured as alternating layer, for example, as shown in figure 1 (b).

After deposition of the layer comprising an electrically conductive coating material, the process may comprise the step of heat treating the coated substrate, preferably wherein the heat treatment comprises heating to at least about 100°C, at least about 125°C or at least about 150°C, for at least about 30 minutes, at least about 45 minutes or at least about 1 hour.

The process may comprise the step of washing the substrate prior to deposition of the layer comprising tin. Washing the substrate may comprise soaking the substrate in water and/or an organic solvent and/or rinsing the substrate with water and/or an organic solvent, preferably washing the substrate comprises soaking the substrate in an organic solvent optionally followed by rinsing the substrate with water. Any commercially available standard laboratory solvent may be used to wash the substrate. Preferably the organic solvent is acetone. After washing, the substrate may be dried.

The layer comprising tin is then deposited on a surface of the substrate. The layer comprising tin may comprise tin as a metal and/or as a tin-alloy. A tin-alloy is preferably a tin-nickel alloy, a tin-antimony alloy, a tin-indium alloy, a tin-gallium alloy, a tin-indium-antimony alloy or tin-nickel-antimony alloy or mixtures thereof. The layer comprising tin may be deposited onto the substrate by electrodeposition. Electrodeposition of the layer comprising tin may be carried out by preparing an aqueous solution of the tin and/or tin alloy, placing the tin solution into contact with the surface of the substrate and applying a current through the tin solution. An aqueous solution of the tin and/or tin alloy may be prepared by dissolving tin salts (for example, tin sulfate or tin chloride) and optionally salts of one or more of nickel, antimony, indium or gallium (for example, sulfates or chlorides) in an aqueous solution. After the electrodeposition of the layer comprising tin is completed, the tin-coated substrate may be washed with (either soaked in or rinsed with) water (preferably deionised water) and the remaining tin on the coating may be wiped off with soft tissue until the clean and metallic coating is observed.

The layer comprising an electrically conductive coating material as described herein is then deposited on said surface of the substrate. Deposition of the electrically conductive coating material may be carried out by screen printing. The electrically conductive coating material may be prepared by combining the carbon-based material, the organic binder and the corrosion inhibitor until a homogeneous mixture is achieved. This coating material may then be screen printed on to the tin-coated substrate using a screen printer. This results in the deposition of a layer comprising an electrically conductive coating material on top of the layer comprising tin. Post-treatment once the coating is prepared is not required. The coated substrate is ready to be used.

## Examples

Reference is now made to the following examples, which illustrate the invention in a nonlimiting fashion.

## EXAMPLE 1

The substrate to which the electrically conductive composite coating is to be applied is copper-cladded composite board and a cross section of the possible coating arrangements is shown in Figure 1.

**Cleaning of the substrate:**

The substrate was soaked in acetone for 5 minutes and then rinsed with deionised water (DI) and dried with a soft tissue.

**Electrodeposition of tin onto the substrate:**

A tin plating solution for electrodeposition was prepared comprising commercially available Sn bath, pH 0.5 - 1 or 0.45 M $SnCl_2.2H_2O$ and 0.45 M $K_4P_2O_7$. The pH was adjusted to 6 with aqueous ammonia solution. The bath temperature during the plating was maintained at 35°C. Plating baths for tin alloys comprises of Sn metal ions with complexing agents (such as chloride or sulfate), with additives and with other metal ions to be alloyed in, for instance Sb, Ga, In or Ni. An example of a plating bath for Sn-6 wt%Sb consists of:

5 g/L $SnSO_4$
5 g/L $Sb(SO_4)_3$
100 g/L concentrated $H_2SO_4$
1 g/L PEG 5800 or 2 g/L PEG 3000
2 g/L Coumarin (7-Diethylamino-4-methylcoumarin)
33 g/L $K_4P_2O_7$

The pH of the above plating bath is around 1 and the bath temperature was maintained at 28°C.

The substrate was copper-cladded composite board and was placed in the tin bath and a current density of -10 mA cm$^{-2}$ was applied for 600 seconds in order to deposit coating with thickness of about 5 $\mu$m, calculated from Faradays law assuming 100% current efficiency and 100% dense Sn deposited.

After the plating was finished, the tin-coated substrate was rinsed with dionised water and the residual tin on the coating was wiped out with soft tissue until the clean tin coating was observed.

**Deposition of electrically conductive coating material using a semi-automatic screen printer:**

A Sunchemical ink was mixed with 1 wt% Benzotriazole until a homogeneous mixture was achieved. To deposit a layer of the electrically conductive coating material, a semi-automatic screen printer was used with blades passing over the surface to be coated four times to achieve a thickness of about 20-25 $\mu$m.

**Heat treatment:**

After the electrically conductive coating material was screen printed, the whole coated article was placed in a preheated oven at about 150°C for 1 hour. After 1 hour, the coated substrate was kept inside the oven and allowed to cool to room temperature.

## EXAMPLE 2

A study was conducted for various coatings and the coatings were tested in an aggressive environment as part of an accelerated corrosion test. A 1 M $Na_2SO_4$ solution adjusted to pH 3 with sulphuric acid at 80°C was selected for these tests. The solution was not deaerated. The potential was stepped to +0.6 V vs. SHE and held at this potential for 1 h. The coatings were continuously immersed in the solution and electrochemical tests, mimicking operation of PEM fuel cell, were conducted. Such tests allowed selection of the best possible coating.

**Coating Selection:**

Three major groups of coatings were tested: the electrically conductive coating material only (max 35 $\mu$m thick), a tin layer coating only (max. about 5 $\mu$m thick), and electrically conductive composite coating comprising a layer comprising an electrically conductive coating material (max. about 35 $\mu$m thick), and a layer comprising tin (max. about 5 $\mu$m thick). These coatings were prepared according to the methods set out in Example 1.

Figure 2 shows results for the three types of coatings tested. It is clear that the corrosion current was related to the type of coating. The electrically conductive coating material (solid curve, figure 2 (a)) showed localised dissolution within 1 hour - large current spikes were observed. This suggests that the coating was degrading with time.

The tin coating on the other hand showed completely different behavior: a large dissolution peak was first observed (dotted curve in figure 2 (b)) and after about 2000 seconds the current rapidly decreased to very low values, then crossed the "time" axis and became cathodic although still increasing to more positive current. This was clear evidence that almost half of the coating dissolved and some oxide layer was formed within 2000 seconds. After this time the dissolution rate was negligible but still increasing with time. Extended accelerated corrosion test showed that after 3.5 days of temperature cycling between room temperature (R.T.) and 80°C, the corrosion current increased from nanoamperes per cm$^{-2}$ to large values, way above the DoE target.

The electrically conductive composite coating (dashed curve in figure 2 (a)), showed the optimum corrosion current: no current spikes present (no local degradation) and the trend of the curve was decreasing with time.

The study of coatings' corrosion was further extended - the time of the testing was much longer and the temperature was cycled between R.T. and 80°C holding at each temperature for a fixed period of time - either 24 or 2 hours - until the failure of the coating was observed (the corrosion current density exceeded DoE target, which is less than about 1 $\mu$A cm$^{-2}$). As before, the test solution used was 1 M $Na_2SO_4$, pH 3. Solutions were not deaerated. The potential was stepped to +0.6 V vs. SHE and held at this potential for 24 h at each temperature.

As mentioned above, the metallic coating lasted only 3.5 days under accelerated corrosion test upon cycling the temperature. For the electrically conductive coating material layer on the other hand, no initial dissolution/oxide formation was recorded but an increasing corrosion current density was seen with time, see Figure 3. After 120 hours, failure was observed.

By combining the metallic and organic coating together to form an electrically conductive composite coating and performing the accelerated long-term corrosion test (cycling the temperature every 2 hours in order to expose the coating to even more harsh conditions), the results were surprisingly good, see Figure 4. After 9 days, there was no significant sign of corrosion increase and the magnitude of the corrosion current was much below the DoE target (DoE target is shown in figure 4). The negative corrosion current density indicates cathodic behaviour of the coating, i.e. the coating is not corroding at this potential. Combination of metallic and organic coating shows a synergistic effect - the coating did not fail within 9 days of accelerated corrosion test - such test would be equivalent to almost 1 year of normal PEM fuel cell operation, whereas single-layered coating deteriorated rather quickly under such conditions.

## EXAMPLE 3 - multilayer coating

In this example, a composite coating comprising two layers comprising an electrically conductive coating material and two layers comprising tin was prepared for much better performance. The composite coating is shown in figure 1 (b). It comprises electrodeposited Sn layer followed by the screen printing of an electrically conductive coating material layer, followed by electrodeposition of Sn-6wt%Sb alloy and final electrically conductive coating material layer. The overall thickness was max. about 40 $\mu$m.

## Corrosion test

The performance of the coating was again tested in the accelerating corrosion test in solution comprising of 1 M $Na_2SO_4$ pH 3 and the temperature was cycled between R.T. and 80°C every 2 h until the corrosion current exceeded the DoE target. The solution was not deaerated. The potential was stepped to +0.6 V vs. SHE and held at this potential for 1 h. The behaviour of the coating is shown in figure 5 - corrosion current density showed a very good behaviour for over 18 days. Although the current density was increased with time, the DoE target was not exceeded.

Clearly, such coating's configuration showed a very good corrosion barrier simultaneously maintaining low contact resistance. In fact, the contact resistance of such family of coatings was below about1 m$\Omega$.cm$^2$.

Embodiments of the invention have been described by way of example only. It will be appreciated that variations of the described embodiments may be made which are still within the scope of the invention.

**Claims**

1. An electrochemical device comprising a coated article comprising;

    a substrate; and
    an electrically conductive composite coating comprising a layer comprising an electrically conductive coating material comprising elemental carbon and an azole-containing corrosion inhibitor; and a layer of tin and/or a binary or ternary alloy of tin with one or more of nickel, antimony, indium or gallium, said tin layer being directly deposited on the substrate and having the electrically conductive coating on top of it.

2. The electrochemical device of claim 1, wherein the electrically conductive composite coating comprises:

    two or more layers comprising an electrically conductive coating material comprising elemental carbon and an azole-containing corrosion inhibitor;
    and
    two or more layers of tin and/or a binary or ternary alloy of tin with one or more nickel, antimony, indium or gallium.

3. The electrochemical device of claim 1 or 2, wherein the electrically conductive coating material comprises elemental carbon comprising carbon black, coal, charcoal, graphite, carbon fibres, carbon nanotubes or graphene or mixtures thereof.

4. The electrochemical device of any preceding claim, wherein the electrically conductive coating material further comprises an organic binder.

5. The electrochemical device of claim 4, wherein the electrically conductive coating material comprises a carbon ink comprising the elemental carbon and the organic binder.

6. The electrochemical device of any preceding claim, wherein the electrically conductive coating material comprises 50 wt% to 90 wt% of the elemental carbon.

7. The electrochemical device of any preceding claim, wherein the azole-containing corrosion inhibitor is benzotriazole, 2-mercaptobenzimidazole, 5-phenyl tetrazole, *bis*[4-amino-5-hydroxy-1,2,4-triazol-3-yl] methane or *bis*[4-amino-5-hydroxy-1,2,4-triazol-3-yl]butane, or mixtures thereof.

8. The electrochemical device of any of claims 4 to 7, wherein the azole-containing corrosion inhibitor is present in the electrically conductive coating material in an amount of 10 wt% or less of the amount of the elemental carbon and the organic binder.

9. The electrochemical device of any preceding claim, wherein the layer or layers of tin and/or binary or ternary tin alloys with one or more of nickel, antimony, indium or gallium contact the substrate.

10. The electrochemical device of any preceding claim, wherein:

    a) the layer comprising an electrically conductive coating material is 1 to 50 $\mu$m thick; and/or
    b) the layer of tin and/or binary or ternary tin alloy with one or more of nickel, antimony, indium or gallium is 1 to 20 $\mu$m thick.

11. The electrochemical device of any preceding claim, wherein the electrically conductive composite coating has an area specific electrical resistance of less than 10 m$\Omega$ cm$^2$.

12. The electrochemical device of any preceding claim, wherein the substrate is a metallic substrate, preferably a copper, iron, titanium, aluminium, nickel or stainless steel substrate.

13. The electrochemical device according to any preceding claim, wherein the substrate is a component in a fuel cell assembly, a battery, a redox flow battery, an electrolyser or a supercapacitor.

14. A process for providing the coated article for the electrochemical device, according to claim 1, the process comprising:

    providing a substrate;
    depositing a layer of tin and/or a binary or ternary alloy of tin with one or more of nickel, antimony, indium or gallium on a surface of the substrate; and
    depositing a layer comprising an electrically conductive coating material comprising elemental carbon and an azole-containing corrosion inhibitor as a layer on said layer of tin and/or a binary or ternary alloy of tin with one or more of nickel, antimony, indium or gallium.

15. The process of claim 14, further comprising:

    depositing a further layer of tin and/or a binary or ternary alloy of tin with one or more of nickel, antimony, indium or gallium on a surface of the coated substrate;
    depositing a further layer comprising an electrically conductive coating material comprising elemental carbon and an azole-containing corrosion inhibitor on said surface of the substrate.

16. Use of the electrically conductive composite coating, according to claim 1, comprising:

> a layer comprising an electrically conductive coating material comprising elemental carbon and an azole-containing corrosion inhibitor; and a layer of tin and/or a binary or ternary alloy of tin with one or more of nickel, antimony, indium or gallium;
> as a corrosion resistant coating for a substrate or a component in an electrochemical device.

**Patentansprüche**

1. Elektrochemische Vorrichtung, umfassend einen beschichteten Gegenstand, umfassend;
ein Substrat; und
eine elektrisch leitende Verbundbeschichtung, umfassend eine Schicht, umfassend ein elektrisch leitendes Beschichtungsmaterial, umfassend elementaren Kohlenstoff und einen Azol enthaltenden Korrosionsinhibitor; und eine Zinnschicht und/oder eine binäre oder ternäre Zinnlegierung mit einem oder mehreren von Nickel, Antimon, Indium oder Gallium, wobei die Zinnschicht direkt auf dem Substrat abgeschieden wird und darauf die elektrisch leitende Beschichtung aufweist.

2. Elektrochemische Vorrichtung nach Anspruch 1, wobei die elektrisch leitende Verbundbeschichtung Folgendes umfasst:

> zwei oder mehr Schichten, umfassend ein elektrisch leitendes Beschichtungsmaterial, umfassend elementaren Kohlenstoff und einen Azol enthaltenden Korrosionsinhibitor; und
> zwei oder mehr Zinnschichten und/oder eine binäre oder ternäre Zinnlegierung mit einem oder mehreren von Nickel, Antimon, Indium oder Gallium.

3. Elektrochemische Vorrichtung nach Anspruch 1 oder 2, wobei das elektrisch leitende Beschichtungsmaterial elementaren Kohlenstoff umfasst, umfassend Ruß, Kohle, Holzkohle, Graphit, Kohlenstofffasern, Kohlenstoffnanoröhren oder Graphen oder Gemische davon.

4. Elektrochemische Vorrichtung nach einem der vorhergehenden Ansprüche, wobei das elektrisch leitende Beschichtungsmaterial ferner ein organisches Bindemittel umfasst.

5. Elektrochemische Vorrichtung nach Anspruch 4, wobei das elektrisch leitende Beschichtungsmaterial eine Kohlenstofftinte umfasst, die den elementaren Kohlenstoff und das organische Bindemittel umfasst.

6. Elektrochemische Vorrichtung nach einem der vorhergehenden Ansprüche, wobei das elektrisch leitende Beschichtungsmaterial 50 Gew.-% bis 90 Gew.-% des elementaren Kohlenstoffs umfasst.

7. Elektrochemische Vorrichtung nach einem der vorhergehenden Ansprüche, wobei der Azol enthaltende Korrosionsinhibitor Benzotriazol, 2-Mercaptobenzimidazol, 5-Phenyltetrazol, *Bis*[4-amino-5-hydroxy-1,2,4-triazol-3-yl]methan oder *Bis*[4-amino-5-hydroxy-1,2,4-triazol-3-yl]butan oder Gemischen davon entspricht.

8. Elektrochemische Vorrichtung nach einem der Ansprüche 4 bis 7, wobei der Azol enthaltende Korrosionsinhibitor in dem elektrisch leitenden Beschichtungsmaterial in einer Menge von 10 Gew.-% oder weniger der Menge des elementaren Kohlenstoffs und des organischen Bindemittels vorliegt.

9. Elektrochemische Vorrichtung nach einem der vorhergehenden Ansprüche, wobei die Zinnschicht(en) und/oder binären oder ternären Zinnlegierungen mit einem oder mehreren von Nickel, Antimon, Indium oder Gallium das Substrat berühren.

10. Elektrochemische Vorrichtung nach einem der vorhergehenden Ansprüche, wobei:

> a) die Schicht, umfassend ein elektrisch leitendes Beschichtungsmaterial, 1 bis 50 $\mu$m dick ist; und/oder
> b) die Zinnschicht und/oder binäre oder ternäre Zinnlegierung mit einem oder mehreren von Nickel, Antimon, Indium oder Gallium 1 bis 20 $\mu$m dick ist.

11. Elektrochemische Vorrichtung nach einem der vorhergehenden Ansprüche, wobei die elektrisch leitende Verbundbeschichtung einen flächenspezifischen elektrischen Widerstand von weniger als 10 m$\Omega$ cm$^2$ aufweist.

12. Elektrochemische Vorrichtung nach einem der vorhergehenden Ansprüche, wobei das Substrat ein metallisches Substrat ist, vorzugsweise ein Kupfer-, Eisen-, Titan-, Aluminium-, Nickel- oder Edelstahlsubstrat.

13. Elektrochemische Vorrichtung nach einem der vorhergehenden Ansprüche, wobei das Substrat eine Komponente in einer Brennstoffzellenanordnung, einer Batterie, einer Redox-Flow-Batterie, einem Elektrolyseur oder einem Superkondensator ist.

14. Verfahren zum Bereitstellen des beschichteten Gegenstands für die elektrochemische Vorrichtung nach Anspruch 1, wobei das Verfahren Folgendes umfasst:

Bereitstellen eines Substrats;
Abscheiden einer Zinnschicht und/oder einer binären oder ternären Zinnlegierung mit einem oder mehreren von Nickel, Antimon, Indium oder Gallium auf einer Oberfläche des Substrats; und
Abscheiden einer Schicht, umfassend ein elektrisch leitendes Beschichtungsmaterial, umfassend elementaren Kohlenstoff und einen Azol enthaltenden Korrosionsinhibitor, als eine Schicht auf der Zinnschicht und/oder einer binären oder ternären Zinnlegierung mit einem oder mehreren von Nickel, Antimon, Indium oder Gallium.

15. Verfahren nach Anspruch 14, ferner umfassend:

Abscheiden einer weiteren Zinnschicht und/oder einer binären oder ternären Zinnlegierung mit einem oder mehreren von Nickel, Antimon, Indium oder Gallium auf einer Oberfläche des beschichteten Substrats;
Abscheiden einer weiteren Schicht, umfassend ein elektrisch leitendes Beschichtungsmaterial, umfassend elementaren Kohlenstoff und einen Azol enthaltenden Korrosionsinhibitor, auf der Oberfläche des Substrats.

16. Verwendung der elektrisch leitenden Verbundbeschichtung nach Anspruch 1, umfassend:

eine Schicht, umfassend ein elektrisch leitendes Beschichtungsmaterial, umfassend elementaren Kohlenstoff und einen Azol enthaltenden Korrosionsinhibitor; und eine Zinnschicht und/oder eine binäre oder ternäre Zinnlegierung mit einem oder mehreren von Nickel, Antimon, Indium oder Gallium;
als eine korrosionsbeständige Beschichtung für ein Substrat oder eine Komponente in einer elektrochemischen Vorrichtung.

**Revendications**

1. Dispositif électrochimique comprenant un article revêtu comprenant ;
un substrat ; et
un revêtement composite électriquement conducteur comprenant une couche comprenant un matériau de revêtement électriquement conducteur comprenant du carbone élémentaire et un inhibiteur de corrosion à teneur en azole ; et une couche d'étain et/ou un alliage binaire ou ternaire d'étain avec un ou plusieurs parmi le nickel, l'antimoine, l'indium ou le gallium, ladite couche d'étain étant directement déposée sur le substrat et ayant le revêtement électriquement conducteur au-dessus d'elle.

2. Dispositif électrochimique de la revendication 1, dans lequel le revêtement composite électriquement conducteur comprend :

deux ou plusieurs couches comprenant un matériau de revêtement électriquement conducteur comprenant du carbone élémentaire et un inhibiteur de corrosion à teneur en azole ; et deux ou plusieurs couches d'étain et/ou un alliage binaire ou ternaire d'étain avec un ou plusieurs nickel, antimoine, indium ou gallium.

3. Dispositif électrochimique de la revendication 1 ou 2, dans lequel le matériau de revêtement électriquement conducteur comprend du carbone élémentaire comprenant du noir de carbone, du charbon, du charbon de bois, du graphite, des fibres de carbone, des nanotubes de carbone ou du graphène ou des mélanges de ceux-ci.

4. Dispositif électrochimique d'une quelconque revendication précédente, dans lequel le matériau de revêtement électriquement conducteur comprend en outre un liant organique.

5. Dispositif électrochimique de la revendication 4, dans lequel le matériau de revêtement électriquement conducteur comprend une encre au carbone comprenant le carbone élémentaire et le liant organique.

6. Dispositif électrochimique d'une quelconque revendication précédente, dans lequel le matériau de revêtement électriquement conducteur comprend 50 % en poids à 90 % en poids de carbone élémentaire.

7. Dispositif électrochimique d'une quelconque revendication précédente, dans lequel l'inhibiteur de corrosion à teneur en azole est le benzotriazole, le 2-mercaptobenzimidazole, le 5-phényl tétrazole, le *bis*[4-amino-5-hydroxy-1,2,4-triazol-3-yl]méthane ou le *bis*[4-amino-5-hydroxy-1,2,4-triazol-3-yl]butane, ou des mélanges de ceux-ci.

8. Dispositif électrochimique de l'une quelconque des revendications 4 à 7, dans lequel l'inhibiteur de corrosion à teneur en azole est présent dans le matériau de revêtement électriquement conducteur en une quantité égale ou inférieure à 10 % en poids de la quantité de carbone élémentaire et de liant organique.

9. Dispositif électrochimique d'une quelconque revendication précédente, dans lequel la couche ou les couches d'étain et/ou d'alliages d'étain binaires ou ternaires avec un ou plusieurs éléments parmi le nickel, l'antimoine, l'indium ou le gallium sont en contact avec le substrat.

10. Dispositif électrochimique d'une quelconque revendication précédente, dans lequel :

a) la couche comprenant un matériau de revêtement électriquement conducteur a une épaisseur de 1 à 50 $\mu$m ; et/ou

b) la couche d'étain et/ou d'alliage binaire ou ternaire d'étain avec un ou plusieurs parmi le nickel, l'antimoine, l'indium ou le gallium a une épaisseur de 1 à 20 $\mu$m.

11. Dispositif électrochimique d'une quelconque revendication précédente, dans lequel le revêtement composite électriquement conducteur a une résistance électrique spécifique de surface inférieure à 10 m$\Omega$ cm$^2$.

12. Dispositif électrochimique d'une quelconque revendication précédente, dans lequel le substrat est un substrat métallique, de préférence un substrat en cuivre, fer, titane, aluminium, nickel ou acier inoxydable.

13. Dispositif électrochimique selon une quelconque revendication précédente, dans lequel le substrat est un composant dans un assemblage de pile à combustible, une batterie, une batterie à flux redox, un électrolyseur ou un supercondensateur.

14. Procédé pour fournir l'article revêtu pour le dispositif électrochimique, selon la revendication 1, le procédé comprenant :

la fourniture d'un substrat ;
le dépôt d'une couche d'étain et/ou d'un alliage binaire ou ternaire d'étain avec un ou plusieurs parmi le nickel, l'antimoine, l'indium ou le gallium, sur une surface du substrat ; et
le dépôt d'une couche comprenant un matériau de revêtement électriquement conducteur comprenant du carbone élémentaire et un inhibiteur de corrosion à teneur en azole comme couche sur ladite couche d'étain et/ou d'un alliage binaire ou ternaire d'étain avec un ou plusieurs parmi le nickel, l'antimoine, l'indium ou le gallium.

15. Procédé de la revendication 14, comprenant en outre :

le dépôt d'une couche supplémentaire d'étain et/ou d'un alliage binaire ou ternaire d'étain avec un ou plusieurs parmi le nickel, l'antimoine, l'indium ou le gallium sur une surface du substrat revêtu ;
le dépôt d'une couche supplémentaire comprenant un matériau de revêtement électriquement conducteur comprenant du carbone élémentaire et un inhibiteur de corrosion à teneur en azole sur ladite surface du substrat.

16. Utilisation du revêtement composite électriquement conducteur, selon la revendication 1, comprenant :

une couche comprenant un matériau de revêtement électriquement conducteur comprenant du carbone élémentaire et un inhibiteur de corrosion à teneur en azole ; et une couche d'étain et/ou d'un alliage binaire ou ternaire d'étain avec un ou plusieurs parmi le nickel, l'antimoine, l'indium ou le gallium ;
comme revêtement résistant à la corrosion pour un substrat ou un composant dans un dispositif électrochimique.

**FIG. 1**

a)

— 102
— 101
— 100

b)

— 102
— 101
— 102
— 101
— 100

**FIG. 2**

a)

b)

FIG. 3

FIG.4

FIG.5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2009061184 A1
- US 2010193573 A1
- US 2005126429 A1
- WO 2013164639 A

**Non-patent literature cited in the description**

- **WIND J ; SPAH R ; KAISER W ; BÖHM G.** Metallic bipolar plates for PEM fuel cells. *J Power Sources,* 2002, vol. 105, 256-260
- **L. LANDIS et al.** Making Better Fuel Cells: Through-Plane Resistivity Measurement of Graphite-Filled Bipolar Plates. Keithly Instruments, Inc, 2002